## Europäisches Patentamt

## European Patent Office

## Office européen des brevets

(11) Publication number: **0 073 219 B2**

(12) **NEW EUROPEAN PATENT SPECIFICATION**

(45) Date of publication of the new patent specification: **19.12.90**

(51) Int. Cl.⁵: **A 61 M 16/00**

(21) Application number: **82900682.4**

(22) Date of filing: **09.03.82**

(86) International application number: **PCT/SE82/00063**

(87) International publication number: **WO 82/03014 16.09.82 Gazette 82/22**

(54) **RESPIRATOR INTENDED FOR CONNECTION TO HUMAN OR ANIMAL AIRWAYS.**

(30) Priority: **10.03.81 SE 8101488**

(43) Date of publication of application:
**09.03.83 Bulletin 83/10**

(45) Publication of the grant of the patent:
**26.06.85 Bulletin 85/26**

(45) Mention of the opposition decision:
**19.12.90 Bulletin 90/51**

(84) Designated Contracting States:
**BE DE FR GB SE**

(56) References cited:
**WO-A-80/01646**
**DE-A-2 831 313**
**SE-B-7 315 392**
**SE-B-7 401 676**
**US-A-4 155 356**

**Anesthesiology, vol. 49, pages 310-318**

(73) Proprietor: **Siemens Elema AB**
**Röntgenvägen 2**
**S-171 95 Solna 1 (SE)**

(72) Inventor: **OLSSON, Sven-Gunnar**
**Postlada 652**
**S-240 17 Södra Sandby (SE)**
Inventor: **JONSON, Björn**
**Nicoloviusväg 11**
**S-223 65 Lund (SE)**

EP 0 073 219 B2

**Beschreibung**

Die Erfindung betrifft einen Respirator zum Anschliessen an die Atemwege von Menschen oder Tieren.

Bei konventioneller Respiratorbehandlung arbeitet man mit Frequenzen und Tidalvolumen, die denen bei einem spontan atmenden Patienten entsprechen. Bei bestimmten Patienten ist es dabei jedoch schwierig, einen zufriedenstellenden Gasaustausch in allen Teilen der Lunge zu erreichen, ohne dass ein schädlicher Druck verwendet wird, der Rupturen verursachen kann oder Blutgefässe derart beeinflusst, dass die Durchblutung der Lungen gehemmt ist.

Um diese schädlichen Drücke zu vermeiden und eine gleichmässigere Ventilierung der Lungen zu erhalten, sind bereits früher gewisse Vorschläge zu Respiratorausführungen gemacht worden.

Ein bekannter Respirator (AGA-Bronchovent, AGA Medical AB, Lidingö, Schweden) besitzt eine Trachealkanüle, die mit einer Abzweigkanüle zum Zuführen von Atemgas unter einem Druck bis zu einem 1 kg/cm² zur Passage der Trachealkanüle für die Durchströmung des Atemgases versehen ist. Die Abzweigkanüle mündet dabei über eine Drosselöffnung, die einen grossen Strömungswiderstand bildet, in die Trachealkanüle. Es wird behauptet, dass dieser Respirator wirksamer ist als übliche Respiratoren, vermutlich dadurch, dass das Atemgas unter Wirbelbildung zugeführt wird, wordurch eine verstärkte Gasdiffusion erreicht wird. Ausserdem wird behauptet, dass er zu einem gesteigerten Schleimtransport führt und die Tendenz für spontane Atmung herabsetzt. Er ist jedoch aufgrund der Anwendung von hohem Druck für das zugeführte Atemgas riskant für den Patienten. Ausserdem ist es schwierig, das zugeführte Atemgas zu befeuchten. Bei diesem bekannten Respirator ist die Atmungsfrequenz bis zu ca. zwei Atemzügen/Sekunde gegenüber normalerweise ca. 1/2—1/4 Atemzug/Sekunde erhöht, was geringere Tidalvolumen und niedrigeren Druck in den Lungen bedeutet. Die verbesserte Ventilierung solite ihren Grund in der entstandenen Wirbelbildung des zugeführten Atemgases haben. Wegen einer genaueren Beschreibung dieses bekannten Respirators wird auf Anesthesia und Analgesia, Vol. 59, No. 8, Aug. 1980, Seite 594—603 verwiesen.

Ein anderer bereits vorgeschlagener Respirator (Klain Jet Ventilator, Acutronic AG, Jona, Schweiz) arbeitet mit sog. "Jet-Injection", wobei der Gasaustausch in den Lungen dadurch zustandekommt, dass diesen pulsweise ein Gasstrahl mit einer Frequenz von bis zu 40 Hz durch eine Passage in de Trachealkanüle oder durch eine kleine Kanüle, die direkt in der Trachea placiert ist, zugeführt wird, während das Ausatmungsgas aus den Lungen durch eine andere Passage in der Tracheralkanüle resp. durch die Trachea abgeleitet wird. Der Respirator gibt einen gleichmässigen und niedrigen Druck in den Lungen, was bei chirurgischen Eingriffen ein Vorteil ist, da keine Bewegungen des Thorax vorkommen und eine minimale Beeinflussung der Blutzirkulation durch die Lungen erreicht wird. Mit einem derartigen Respirator sind jedoch viele Nachteile verbunden. Der Gasstrahl kann die Schleimhäute in der Trachea und die Flimmerhaare beschädigen und im schlimmsten Fall die Schleimhäute penetrieren. Es ist schwer, das zugeführte Atemgas zu befeuchten und es ist nicht möglich, durch Beobachtung der Bewegung des Brustkorbes zu kontrollieren, ob eine Ventilierung geschieht. Man kann in diesem Fall nicht auf einfache Art und Weise bestimmen, wieviel Atemgas in den Lungen war oder die Zusammensetzung des Gases in den Lungenbläschen messen. Betreffend dieses Respirators wird auf Crit. Care Med. 5:280—287, 1977 verwiesen.

Schliesslich ist ein Respirator vorgeschlagen, bei dem die Trachealkanüle mit einer diese kreuzenden Röhre versehen ist, durch welche kontinuierlich Atemgas geleitet wird. Ein Pulsator, der quer zu dem Gasstrom arbeitet, sorgt für den Gastransport durch die Passage der Trachealkanüle mit einer Frequenz bis zu 40 oder 50 Hz. Soweit bekannt, ist dieser Respirator bisher nur versuchsweise bei Tieren und nicht bei Menschen angewendet worden. Dabei konnte ein erthöhter Schleimtransport festgestellt werden, jedoch ist es unmöglich, die Ventilationseffekte zu kontrollieren. Die Auswirkungen der hohen Frequenzen auf die Blutzirkulation und biologische Substanzen ist unbekannt. In diesem Zusammenhang wird auf Proc. Am. Soc. Exp. Biol. 38 (part II): 951, 1979, und auf Science, Vol. 209, 609 und 610, 1980, verwiesen.

Ausser den oben angegebenen Nachteilen weist eine längerwährende hochfrequente Ventilation, wie sie beispielsweise bei der "Jet-Injection" oder der "Pulsatormethode" vorkommt, den weiteren Nachteil auf, dass die Funktion der Lungen verschlectert wird. Beispielsweise werden die Lungen steifer und die Sauerstoffaufnahme des arteriellen Blutes schlechter, wodurch sich in den Lungensäcken Flüssigkeit ansammeln kann.

Darüber hinaus ist zwar aus der US—A— 4 155 356 ein Respirator zum Anschliessen an die Atemwege von Menschen oder Tieren bekannt, der eine erste Anordnung zur Zufuhr von Atemgas an die Atemwege und Abgase von Atemgas von den Atemwegen zur Ventilierung der Lunge, eine zweite Anordnung mit einer Leitung zur Zufuhr von Druckgas an ein Ventil zur separaten, gepulsten Gaszufuhr zu den Atemwegen unabhängig von dem von der ersten Anordnung verursachten Atemfluss, sowie eine Steuereinheit für die Zweite Anordnung zur Festlegung mindestens einer charakteristischen Grösse der Gasimpulse aufweist. Die Absicht mit diesem Respirator ist es, einem Kollaps der Atemwege während der Exspiration entgegenzuwirken. Dazu wird den Atemwegen nur während der Exspirationsphase ein Gas, nämlich Luft, impulsweise zugeführt. An eine Verbesserung des Gasaustausches in allen Teilen de Lunge ist dabei nicht gedacht.

Primäres Zil der vorliegenden Erfindung ist es, einen Respirator zum Anschliessen an die Atemwege bei Menschen oder Tieren zu schaffen, der den Patienten auf konventionelle Weise ventilieren kann und der ausserdem optimal und sicher die Möglichkeit schafft, wahlfrei die eine oder andere der neuen Techniken, die man bei den oben beschriebenen Ventilationsprinzipien versucht hat anzuwenden, einzusetzen, und der ausserdem die Möglichkeit der Kombination von älteren und neueren Techniken schafft. Damit wird bezweckt, einen Respirator zu schaffen der eine adäquate und passend verteilte alveoläre Ventilierung der Lungen des Patienten ermöglicht, wobei die Ventilierung in Beziehung auf Volumen und Zusammensetzung des Atemgases kontrollierbar sein soll, der weiterhin mit kleinen tidalen Atmungsbewegungen, d.h. mit wünschenswert kleinen Atmungsvolumina und damit verbundenem niedrigen Druck in den Atemwegen arbeitet, so dass die Lungen und die Luftwege, speziell bei Patienten mit steifen Lungen, geschützt sind, und mit dem nur geringe Einwirkungen auf Blutgefässe und damit verbundene Hemmungen der Durchblutung der Lungen verbunden sind, ohne dass die Nachteile der bekannten Respiratoren in Kauf genommen werden müssen.

Ein weiteres Ziel ist es, einen Respirator zu schaffen, bei dem Gase in spezieller Zusammensetzung und genau quantifizierbarer Weise dem Atemgas zugesetzt werden können (die Zufuhr kann in bezug auf das Volumen und den Zeitpunkt bestimmt werden), so dass man durch eine Analyse des ausgeatmeten Atemgases Schlüsse über die Funktionsweise der Lungen ziehen kann.

Weiterhin ist es ein Ziel der Erfindung, eine genaue Dosierung der Zuführung von therapeutischen oder sonst pharmakologisch aktiven Substanzen betreffend der Menge resp. des Zeitpunktes im Atemzyklus zu ermöglichen. Beispielsweise kann Narkosegas dem Atemgas pulsweise zugeführt werden, wobei die Impulse eine höhere Konzentration von Narkosegas enthalten können als normalerweise für Atemgase zulässig ist. Nachdem die Konzentration des Narkosegases im Blut proportional der Konzentration in dem ausgeatmeten Atemgas ist, kann die Dosierung daher leicht durch Messen der zuletzt genannten Konzentration im Alveolärgas gesteuert werden, wenn dieses während der Ausatmung die Luftwegsöffnungen erreicht hat, indem in Abhängigkeit davon die Narkosegasimpulse im Einatmungsgas angepasst werden.

Ein weiteres Ziel der Erfindung ist es, sich der Möglichkeit zum Messen der alveolären Gaskonzentration zu versichern, auch wenn die genannten neuen Techniken für das Fördern des Gasaustausches in den Lungen zugelassen werden.

Schliesslich soll durch die Erfindung ein Respirator geschaffen werden, bei dem zum Schluss einer Ausatmung das ausgeatmete Gas in der Trachealkanüle resp. der Anschlussleitung zum Y-Stück des Ventilators mit frischen oder unverbrauchten Beatmungsgas gespült werden kann, um zu verhindern, dass das benutzte Atemgas erneut in die alveolären Räume des Patienten gelangt.

Zu diesem Zweck weist der Respirator gemäss der Erfindung die Kennzeichen auf, die aus dem Patentanspruch 1 hervorgehen.

Die Erfindung soll im folgenden unter Bezugnahme auf die beigefügten Zeichnungen näher erläutert werden, wobei

Fig. 1 ein Zusammenstellungsschema über den Respirator gemäss der Erfindung,

Fig. 2 ein schematisches bild der in den Respirator einmündenden Anordnungen zur ventilgeregelten separaten Zufuhr von Gas zu den Atemwegen und

Fig. 3 ein Schaltbild über das System zum Steuern der Ventile der Anordnung gemäss Fig. 2 ist.

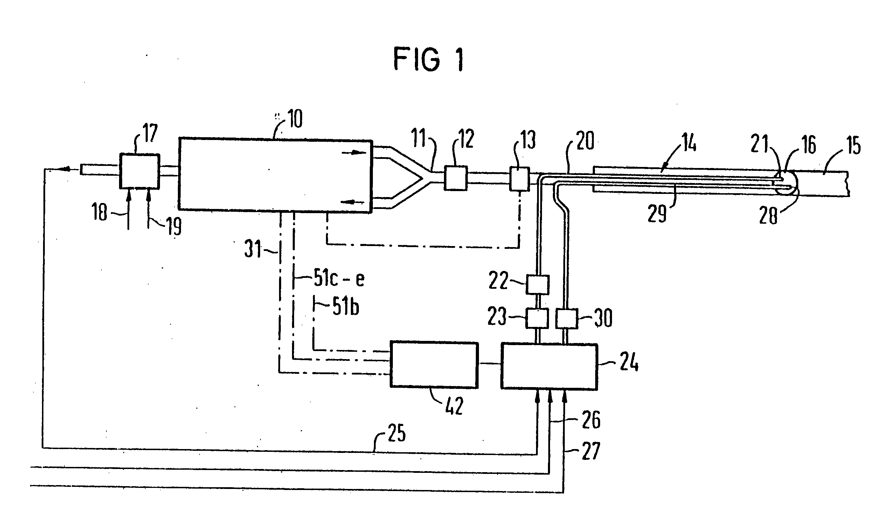

Wie die schematische Fig. 1 zeigt, ist ein Lungenventilator 10, beispielsweise ein Servoventilator vom Typ 900 B de Siemens-Elema AB, Solna, Schweden, an ein Y-Rohr 11 angeschlossen, das über einen Befeuchter 12, beispielsweise einen Servobefeuchter 150 der Siemens-Elema AB, und einen Rezeptor 13 für einen Gasanalysator, beispielsweise vom Typ 930 der Siemens-Elema AB, mit einer Trachealkanüle 14 verbunden ist. Die Trachealkanüle ist zum Einführen in die Trachea (Luftröhre), die mit 15 angedeutet ist, vorgesehen, und kann an einem Ende—dem, das in die Luftröhre eingeführt ist— mit einer expandierbaren Manschette 16 bekannter Ausführung zum Abdichten zwischen der Aussenseite der Trachealkanüle und der Innenseite der Luftröhre versehen sein. Es ist jedoch nicht notwendig, dass eine derartige Abdichtung zustandekommt. Die Trachealkanüle kann auch relativ lose in die Luftröhre eingeführt sein. Weiterhin soll erwähnt werden, dass die Trachealkanüle durch einen Schlauch ersetzt sein kann, der den Lungenventilator 10 mit einer Beatmungsmaske verbindet, die von konventioneller Art und dazu vorgesehen ist, über der Nase und dem Mund eines Patienten appliziert zu werden. Der Lungenventilator arbeitet in bekannter Weise zum Zuführen und Ableiten von Atemgas zu resp. von den Atemwegen über die Trachealkanüle 14 zum Ventilieren der Lungen. Das Atemgas wird dem Lungenventilator von einem Mischer 17 für Atemgas zugeführt, der teilweise an eine Leitung 18 für reinen Sauerstoff ($O_2$) und teilweise an eine Leitung 19 für Luft oder Lachgas angeschlossen ist.

In der Trachealkanüle 14 ist eine Passage oder Leitung 20 angeordnet, die abgegrenzt ist von der übrigen Passage durch die Trachealkanüle, d.h. von der Passage für Atemgas vom Lungenventilator 10. Diese Leitung 20 mündet am inneren Ende der Trachealkanüle bei 21. Die Leitung ist über einen Befeuchter 22 und ein Sterilfilter 23 an eine Anordnung 24 für die ventilgeregelte separate Zuführung von Gas zur Leitung 20 unabhängig vom Fluss des Atemgases, der durch die Trachealkanüle strömt und vom Lungenventilator 10 gesteuert ist, jedoch in Koordination mit der zuletzt genannten Strömung und/oder den Beatmungsbewegungen bei einem Patienten, der mit dem Respirator behandelt wird, angeschlossen. Die Anordnung 24 ist über eine Leitung 25 an den Gasmischer 17 angeschlossen, sie ist jedoch auch an Leitungen 26 und 27 angeschlossen und kann darüber hinaus an eine gewünschte Anzahl weiterer Leitungen zur Zufuhr von Atemgas mit anderem Druck als dem des Atemgases vom Lungenventilator und zur Zuführ von verschiedenen Gasen anderen Art als Atemgas, beispielsweise Testgas, Narkosegas oder Gas mit therapeutischer Wirkung angeschlossen sein. Die Anordnung 24 enthält Mittel, um die Zufuhr von Gas durch die Leitung 20 zu regeln, beispielsweise um diesen Gasstrom zu pulsen. Diese Regelung kann, wie nachfolgend beschrieben, auf unterschiedliche Weise und in Abhängigkeit von unterschiedlichen Regelgrössen geschehen.

Am inneren Ende der Trachealröhre 14 ist ein Druckeinlass 28 angeordnet, der mit einer Passage oder Leitung 29 in der Trachealkanüle kommuniziert und ebenso wie die Leitung 20 von der übrigen Passage durch die Trachealkanüle abgegrenzt und über ein Sterilfilter 30 an die Anordnung 24 zu deren Steuerung in Abhängigkeit vom Druck am inneren Ende der Trachealkanüle angeschlossen ist. Weiterhin ist die Anordnung 24 über eine elektrische Verbindung an den Lungenventilator 10 angeschlossen, um die Anordnung 24 in Abhängigkeit von der Funktion des Lungenventilators bei gleichzeitiger Koordinierung der von der Anordnung 24 geregelten Gaszufuhr mit dem Atemfluss des Lungenventilators resp. den Atembewegungen des Patienten zu steuern, wie im folgenden näher beschrieben wird.

Die Mündung 21 zur Zufuhr von Gas von der Anordnung 24 kann ebenso wie der Druckeinlass 28 in dem Schlauch zu einer Atemmaske oder der Trachealkanüle konventioneller Ausführung oder an beliebiger Stelle in der Passage für den Atemgasfluss angeordnet sein, vorzugsweise jedoch so nahe den Lungen des Patienten wie möglich.

Es können Mittel zur Zufuhr von pharmakologisch aktiven Substanzen zu dem durch die Leitung 20 strömenden Gas vorgesehen sein, beispielsweise in Form von Düsen zur Zufuhr derartiger Substanzen in flüssiger Form.

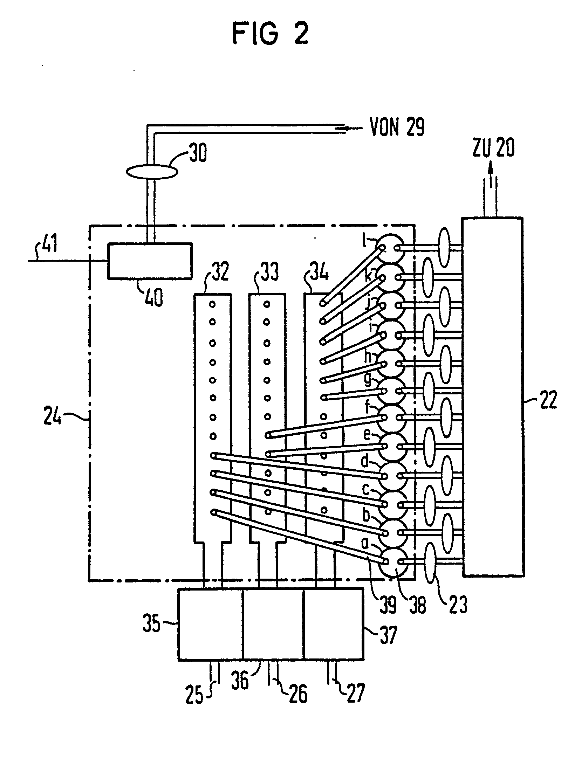

Wie die Anordnung 24 gesteuert wird, wird weiter unten näher erklärt. Zuerst soll jedoch der konstruktive Aufbau der Anordnung 24 unter Bezugnahme auf die Fig. 2 beschrieben werden.

Die Leitungen 25, 26 und 27 sind jede über einen Druckregulator 35, 36 und 37 an einen Verteiler 32, 33 und 34 angeschlossen. Unter Umständen können diese Druckregulatoren weggelassen werden, wenn Gasquellen mit einem passenden Druck zur Verfügung stehen. Eine Anzahl Miniatursolenoidventile 38a—38l sind vorgesehen. Diese Ventile können beispielsweise vom Typ LIF LF AA 1200118H von "The Lee Company", Westbrooke, Connecticut, USA, sein. Die Ventile sind über Schläuche 39 mit Schnellkupplungen an die Verteiler angeschlossen, wobei die Ventile 38a—38d an den Verteiler 32, die Ventile 38e und 38f an den Verteiler 33 sowie die Ventile 38g bis 38l an den Verteiler 34 angeschlossen sind. Es besteht aber auch die Moglichkeit, die Ventile in einer anderen Konfiguration anzuschliessen, so dass eine willkürliche Anzahl Ventile an einen gewünschten Verteiler angeschlossen sein kann. Anstelle von Schläuchen 39 mit Schnellkupplungen können permanente Verbindungen mit oder ohne Mehrwegeventilen zwischen den Solenoiden 38a—38l und den Verteilern 32, 33 und 34 angeordnet sein. Es ist auch möglich, dass sämtliche Anschlüsse jeden Verteilers mit Solenoidventilen versehen sind, so dass ein Umkuppeln nicht notwendig ist. Jedes Solenoidventil hat ein individuelles Sterilfilter 23, über das es mit dem Befeuchter 22 verbunden ist.

Die Verbindung jeder Leitung 25, 26, 27 über zwei oder mehrere Miniatursolenoidventile mit der Leitung 20 dient einem dreifachen Zweck: Erstens kann der Gasfluss von jeder einzelnen Leitung 25, 26 oder 27 zur Leitung 20 geregelt werden, indem ein oder mehrere der an die betreffende Leitung 25, 26 oder 27 angeschlossenen Ventile geöffnet werden, zweitens kann der Gasfluss von einer Leitung 25, 26 oder 27, vorausgesetzt, dass der Gasdruck sehr hoch ist im Verhältnis zum Druck in der Leitung 20, schnell gestartet und wieder gestoppt werden, um Impulse mit einer sehr hohen Frequenz zu erzeugen—in der Praxis bis zu 100 Hz—und um eine schnelle Regelung des Flusses resp. Druckes dadurch zu ermöglichen, dass die beweglichen Teile in diesen Miniatursolenoidventilen geringe Masse haben, und drittens kann das Risiko eliminiert werden, dass ein Patient zu Schaden kommen kann, wenn ein einzelnes Ventil mechanisch oder elektrisch klemmen sollte. Was den letzten Punkt betrifft, so wird das genannte Ziel dadurch erreicht, dass die Durchlasspassage in jedem Miniatursolenoidventil eng ist, so dass nur ein begrenzter Fluss passieren kann. Das bedeutet, dass sich nicht schnell ein hoher Druck im Patienten aufbauen kann, wenn ein Ventil in der offenen Lage verbleibt, wenn es eigentlich geschlossen sein sollte. Dadurch wird Zeit gewonnen, damit vorhandene Sicherheitsanordnungen im Lungenventilator in Funktion treten und eine Passage zum Aussenraum öffnen und das verantwortliche Personal für die Respiratorbehandlung alarmieren können. Andererseits erhält man einen Gasfluss, auch wenn ein Ventil von zwei oder mehreren parallel gekoppelten Ventilen in geschlossener Lage verbleiben sollte, wenn es eigentlich geöffnet sein müsste.

Die Ventile 38 ermöglichen es, dass Gase von den Leitungen 25, 26 und 27 unabhängig voneinander in gewünschter Menge, während gewünschter Dauer und zu gewünschtem Zeitpunkt zugeführt werden. Sie ermöglichen es auch, dass diese Gase in gewünschten Proportionen gemischt werden.

Die Leitung 29 ist über ein Sterilfilter 30 an einen Druckgeber 40 zur Abgabe eines elektri-

schen Signales an die Leitung 41 angeschlossen, wobei dieses Signal proportional dem bei 28 abgefühlten Druck ist. Der Druckgeber kann vom Typ 63 95 628 E037E von Siemens-Elema AB sein.

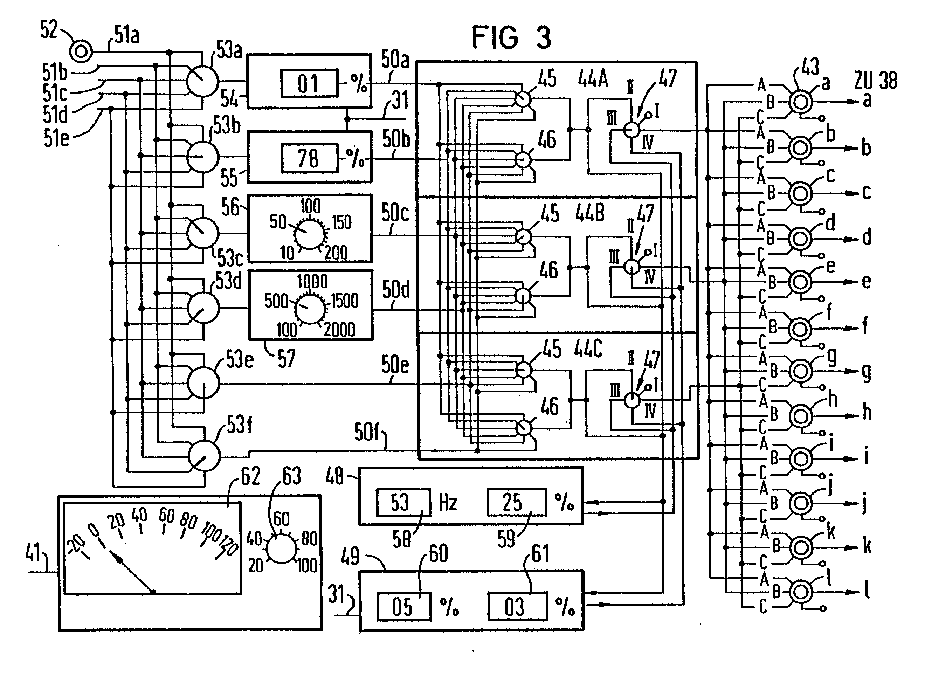

Wie Fig. 1 zeigt, ist an die Anordnung 24 eine Steuereinheit 42 angeschlossen. In dieser Steuereinheit, die detaillierter in Fig. 3 dargestellt ist, ist jedes einzelne Miniatursolenoidventil 38 an einen individuellen Umschalter 43a bis 43l angeschlossen. Jeder derartiger Umschalter ermöglicht den Anschluss der zu den Solenoidventilen gehörenden Spulen an eine von drei Programmwerken 44 A bis 44C, was in der Fig. 3 mittels der Leitungen A, B, C gezeigt ist. Jeder Umschalter hat eine Nulllage, mit O bezeichnet, in welcher die Ventile abgeschaltet sind.

Jedes Programmwerk 44A bis 44C hat Organe 45 und 46 für die Wahl von Start und Stoppsignal und ausserdem einen Wähler 47 mit vier Lagen, bezeichnet I bis IV. In der Lage I sind die durch Einstellung des Umschalters 43 an das betreffende Programmwerk angeschlossenen Ventile 38 als Gruppe abgeschaltet, während diese Ventile in der Lage II kontinuierlich aktiviert sind zwischen den Zeitpunkten, die durch die Start- und Stoppsignale der Organe 45 und 46 bestimmt sind. In der Lage III sind sie an einen Impulsgeber 48 angeschlossen, über den sie pulsweise zwischen Start- und Stoppzeitpunkten aktiviert werden, und in der Lage IV sind sie an einen Impulsgeber 49 angeschlossen, um von diesem zwischen Start- und Stoppzeitpunkten pulsweise aktiviert zu werden.

Die Organe 45 und 46 ermöglichen es, dass die zugehörigen Programmwerke an jede beliebige von sechs Leitungen 50a—50f anschliessbar sind, denen Steuersignale in Form von Impulsen über Leitungen 51a—51e zugeführt werden. Dabei ist die Leitung 51a an ein manuell betätigbares Organ 52 für die manuelle Abgabe eines Signalimpulses und die Leitung 51b an einen externen Apparat zur Abgabe von Signalimpulsen, beispielsweise ein Signalgeber zur Abgabe von Signalen synchron zur Herzfrequenz des Patienten, angeschlossen. Die übrigen Leitungen 51c—51e sind an den Lungenventilator 10 angeschlossen, wie es auch in Fig. 1 angedeutet ist. Bei dem als Beispiel gewählten Lungenventilator Typ Siemens-Elema AB, Servoventilator 900B, wird der Leitung 51c ein Signalimpuls zugeführt, der den Start der Einatmungsphase (Inspiration) repräsentiert, der Leitung 51d ein Signalimpuls, der den Start der Pause zwischen Einatmungs- und Ausatmungsphase repräsentiert, und der Leitung 51e schliesslich ein Signalimpuls, der den Start der Ausatmungsphase (Exspiration) repräsentiert.

Die Signale auf den Leitungen 51a—51e können den Programmwerken 44A, 44B und 44C auf unterschiedliche Art und Weise zugeführt werden, wozu sechs Wähler 53a—53f vorgesehen sind, von denen jeder über fünf Eingängen an die entsprechenden Leitungen 51a—51e angeschlossen ist.

Die Wähler 53a und 53b sind an je einen Verzögerungskreis 54 resp. 55 angeschlossen, denen beiden Taktimpulse über eine Leitung 31, siehe auch Fig. 1, vom Servoventilator 10 zugeführt werden und die an die Leitungen 51a resp. 50b angeschlossen sind. Die Taktimpulse auf der Leitung 31 unterteilen einen Atemzug, d.h. einen Ventilationszyklus, bestehend aus Einatmungsphase (Inspiration). Pause und Ausatmungsphase (Exspiration), in 100 gleiche Teile oder Intervalle, wobei man mit einem Stellwerk (Daumenrad) eine Verzögerung von 0—99% eines Atemzyklus einstellen kann. In Fig. 3 ist am Verzögerungskreis 54 eine Verzögerung von 1% und am Verzögerungskreis 55 eine Verzögerung von 78% eingestellt.

Die Wähler 53c und 53d sind auf analoge Weise an Verzögerungskreise 56 resp. 57 angeschlossen, wobei der Verzögerungskreis 56, der an die Leitung 50c angeschlossen is das Einstellen einer Verzögerung von 10 bis 200 ms und der Kreis 57, der an die Leitung 50d angeschlossen ist, das Einstellen einer Verzögerung von 100—2000 ms ermöglicht. In Fig. 3 werden die zuletzt genannten Verzögerungskreise auf 50 resp. 500 ms eingestellt gezeigt. Schliesslich ist es möglich, eine direkte Verbindung zwischen den Leitungen 51a—51e und den Leitungen 50e resp. 50f mit Hilfe der Wähler 53e resp. 53f herzustellen.

Der Impulsgeber 48 hat ein Einstellwerk 58 (Daumenrad)] zur Einstellung der gewünschten Frequenz zwischen 1—99 Hz (dargestellt ist eine Einstellung für 53 Hz) und ein Einstellwerk 59 (Daumenrad) zur Einstellung des Anteiles jeder Periode, der aktiv ausgenutzt werden soll (dargestellt ist die Einstellung 25%). Der Impulsgeber 49 ist an die Leitung 31 angeschlossen, die zum Lungenventilator 10 geht—siehe Fig. 1—, und hat ein Einstellwerk 60 (Daumenrad) zum Einstellen einer Periodenlänge von der in dem Servoventil eingestellten Periodlänge de Ventilationszyklen (eingeteilt in 100 Teile) sowie ein Einstellwerk 61 zur Auswahl davon, wieviele Teile der eingestellten Periodenlänge aktiv ausgenutzt werden sollen. Da es sich um hundert Teile handelt, wird die Einstellungt in Prozenten angegeben. In Fig. 3 ist die Einstellung an Beispielen von 5% für das Einstellwerk 60 und 3% für das Einstellwerk 61 erläutert, was bedeutet, dass von den fünf Teilen, die mit dem Einstellwerk 60 gewählt wurden, nur drei Teile aktiv ausgenutzt werden, was mit dem Einstellwerk 61 gewählt worden ist.

Schliesslich umfasst die Steuereinheit 42 ein an die Leitung 41 angeschlossenes Messinstrument 62 zur Anzeige des gemessenen Druckes und ein Stellwerk 63 für die Wahl des maximal zulässigen Druckes. Es sind Mittel (nicht dargestellt) zum Abschalten sämtlicher Miniatursolenoidventile oder so vieler dieser Ventile vorgesehen, dass der eingestellte Druck nicht nennenswert überschritten wird. Das geschieht mit einer Verzögerung von weniger als 5 ms.

Mit der beschriebenen Steuereinheit 42 ist es möglich, die Gaszufuhr zur Leitung 20 teils mit Hinsicht auf die Zusammensetzung des Gases und teils mit Hinsicht auf die Frequenz der abgegebenen Gasimpulse, deren Zeitpunkt im Atmungszyklus sowie deren Länge und Form im Hinblick auf den Gasdurchfluss und/oder Druck zu steuern sowie ausserdem eine Koordinierung der Gasimpulsabgabe mit der Funktion des Lungenventilators 10 zu ermöglichen. In diesem Zusammenhang soll erwähnt werden, dass Mittel zur Anzeige der Gasmenge, die der Leitung 20 von jeder Leitung 25, 26, 27 zugeführt wird, vorgesehen sein können. Nachdem die Durchströmungsmenge pro Zeitenheit (Fluss) jedes Miniatursolenoidventils aufgrund dessen bekannt ist, dass der Druckabfall am Ventil hoch ist im Verhältnis zum Druck in der Leitung 20, kann das Gasvolumen durch Integration der Offenhaltungszeiten für die Anzahl Ventile, die gleichzeitig aktiviert werden, bestimmt werden.

Die Zufuhr des Einatmungsgases zu den Lungenbläschen ist die Voraussetzung für einen Gasaustausch mit dem Blut in den Lungen. Der Transport von Frischgas zu den Lungenbläschen geschieht von de Luftwegsöffnung mittels Konvexion durch den Bronchialbaum. Wenn dieser sich immer mehr verzweigt, wächst die gesamte Querschittsfläche und die Transportgeschwindigkeit durch Konvexion nimmt schell ab. Dieses in der Peripherie der Lungen besonders kräftige Anwachsen der besagten Querschnittsfläche schafft die Grenzzone zwischen dem eingeatmeten Frischgas und altem alveolären Gas. Am weitesten aussen in den Lungen geschieht der Transport des eingeatmeten Gases praktisch genommen nur durch Diffusion über diese Grenzzone.

Bei konventioneller Respiratorbehandlng wird das eingeatmete Atemzugvolumen nur unvollständsig ausgenutzt, da das Gas, das bereits während des vorangegangenen Atemzuges am Gasautausch teilgenommen hat und während der Ausatmung die Luftwege und die Trachealkanüle gefüllt hat, zu Anfang der Einatmung wieder zu den Lungenbläschen geführt wird. Der Teil des eingeatmeten Frischgases, der weit in die Lungen hineingelangt, mischt sich dabei durch Diffusion unvollständig mit alveolärem Gas, was zusätzlich die Ausnutzung des eingeatmeten Gasvolumens begrenzt.

Ein Respirator gemäss der vorliegenden Erfindung erlaubt einen deutlich gesteigerten Ausnutzungsgrad des eingeatmeten Gases dadurch, dass zwei getrennte Zuführungswege und unterschiedliche Art und Weisen für die Zufuhr des einzuatmenden Gases ausgenutzt werden. Dadurch, dass über die Leitung 20 während der Ausatmung (Exspiration) Gas zugeführt wird, kann die Trachealkanüle und der Luftweg bishin zum Y-Rohr 11 frei von früher ausgenutztem Gas gespült werden. Diese Zufuhr über die Leitung 20 kann gerichtet und gepulst sein, so dass sogar wesentliche Teile der Luftwege des Patienten frei von ausgenutzem Gas gespült werden können. Am Ende der Ausatmung kan zur Förderung der totalen Ventilierung eines Patienten gleichfalls ein Austusch des alvaeolären Gases geschehen.

Die Zufuhr von Gas durch die Leitung 20 kann weiterhin während der Ausatmung geregelt werden, dami der während der Ausatmung vorgesehene Druck in den Luftwegen aufrechterhalen werden kann. Dieser Druck wird vorteilhafterweise übver die Leitung 29 mit dem Druckaufnehmer 40 gemessen. Der vorgesehene Druck während der Ausatmung kann weiterhin zum Aufrechterhalten eines für die beste Lungenfunktion passenden Lungenvolumens geregelt werden. Lungenvolumen und Lungenfunktion kann bei der Respiration gemäss vorliegender Erfingung in der unten beschriebenen Art und Weise gemessen werden.

Die Mischung des Einatmungsgases mit alveolärem Gas über Diffusion kann durch die Erfindung gefördert werden, indem ein pulsierender Fluss durch die Leitung 20 zugeführt wird, wodurch das Gas in den Lungen zum Oszillieren gebracht wird, was zu einer verbesserten (forcierten) Diffusion führt. Die Gasimpulse können während der Ausatmung im Hinblick auf Frequenz, Fluss und Druck derart geformt sein, dass sie auf beste Art und Weise die Luftwege ausspülen, während die Gasimpulse bei der Einatmung derart geformt sind, dass sie auf beste Art und Weise zu einer verbesserten Diffusion führen.

Durch die Verbesserung des Grades, in dem das Gas in jedem Atemzug auf beschriebene Weise ausgenutzt wird, kann ein ausreichender Gasaustausch in den Lungen mit einem geringeren Atemzugvolumen und damit niedrigerem Druck und weniger schädlichen Einwirkungen auf die Lungen durch die Respiratorbehandlung erreicht werden. Weiterhin werden damit die erwähnten schädlichen Einwirkungen auf das Epithel der Luftege vermieden, die bei der Ventilierung der Lungen allein mit Gaspulsen mit hoher Frequenz und hohem Energieinhalt in der Trachea entstehen.

Die Analyse des Gases aus den Lungenbläschen zur Steuerung der Ventilierung ist gemäss der Erfindung dann möglich, wenn am Ende einer einzelnen Einatmung aller Gasfluss durch die Leitung 20 unterbrochen wird, so dass unvermischtes Gas von den Lungenbläschen bei der anschliessenden Ausatmung den an den Rezeptor 13 angeschlossenen Analysator erreicht.

Der Nachteil, dass bei langwieriger Ventilierung allein mit hoher Atmungsfrequenz Teile der Lungen zusammenfallen und damit das Blut nur unvollständig mit Sauerstoff versorgt wird, wird bei der Respiration gemäss der Erfindung dadurch vermieden, dass die Lungen einer ausreichend langsamen Atemzugfrequenz kombiniert mit schnelleren Gasimpulsen ausgesetzt werden derart, dass der Aussenfilm in den Lungenbläschen durch seine zeitabhängige Hysterese im Hinblick auf die Aussenspannung kontra Volumen der Lungenbläschen die Energie aufnimmt, die zur Aufrechterhaltung einer stabilien und gleichmässigen Expansion der Lungen erforderlich ist.

Eine schnelle Anpassung der Funktionsart des Respirators gemäss der Erfindung an den Druck in den Luftwegen kann durch eine Kombination von Charakteristika bei der Respiratorkonstruktion erzielt werden. Die Transmissionszeit für Druck- und Flussimpulse kann dadurch niedrig gehalten werden, dass die geringes Volumen erfordernde Anordnung 24 nahe bei der Änderung plaziert wird. Die Verzögerung bei der Änderung der Funktionsweise wird durch die grosse Schnelligkeit der mechanischen Komponenten (Miniatursolenoidventile 38) in der Anordnung 24 sehr klein. Diese Eigenschaften sind u.a. wertvoll, wenn der Respirator der Atmung eines Patienten, der spontan mit Unterstützung des Respirators atmet, gut folgen soll.

Ausser der Möglichkeit, auf konventionelle Weise Narkosegas über den Lungenventilator 10 zuzuführen, ermöglichkeit, auf konventionelle Weise Narkosegas über den Lungenventilator 10 zuzuführen, ermöglicht der Respirator gemäss der Erfindung, wie bereits erwähnt, die Möglichkeit, das Narkosegas über die Leitung 20 zuzuführen. Der Fluss des Narkosegases durch diese Leitung kann dadurch gesteuert werden, dass eine bestimmte Anzahl Miniatursolenoidventile 38 in der Anordnung 24 geöffnet werden. Das Volumen des zugeführten Narkosegses kann ausserdem durch Steuern der Zeiten für die Zufuhr von Narkosegase geregelt werden. Dadurch, dass das Narkosegas allein während einer bestimmten Phase, beispielsweise unmittelbar zu Beginn de Einatmung zugeführt wird, ergibt sich der Vorteil, dass alles Narkosegas die Lungenbläschen erreicht. Auf diese Weise kann die Menge des Narkosegases, die den Lungenbläschen zugeführt wird und damit dem Blut, exakt gesteuert werden. Der Vorteil damit ist, dass alles Narkosegas ausgenützt wird. Für den Fall, dass der an den Rezeptor 13 angeschlossene Gasanalysator den Narkosegasgehalt messen kann, kann auf die gleiche Weise, wie bereits betreffend der Messung von Alveolärgas beschrieben, auch der Narkosegasgehalt im Alveolärgas bestimmt werden. Da der Rezeptor 13 während der Einatmung frei von Markosegas gespült wird, kann de Analysator während dieser Periode auf Null zurückgestellt werden, wodurch die Notwendigkeit besonderer Anordnungen und Massnahmen für dessen Nullstellung eliminiert werden. Die das Narkosegas betreffenden Aussagen haben auch Gültigkeit für Gas mit einer anderen vorgesehenen Wirkung als Narkose, z.B. Gase mit therapeutischer Wirkung.

Mit dem Respirator gemäss der Erfindung kann die Funktion der Lungen und der Zirkulationsorane detailiert studiert werden. Durch die Leitung 20 kann Testgas mti Präzision im Hinblick auf den Zeitpunkt im Atmungszyklus und im Hinblick auf das Volumen zugeführt werden. Es wird vorausgesetzt, dass der an den Rezeptor 13 angeschlossene Analysator die Gase, die in den Testgasen enthalten sind, analysieren kann. Durch Bestimmung der Verdünnung einer vorgegebenen Menge Testgases, das sich nicht in physiologischen Flüssigkeiten löst, beispielsweise Schwefelhexafluoarid, $SF_6$, kann das Volumen des alveolären Gases bestimt werden. Wenn das Testgas ausserdem Kohlenmonoxid, CO, enthält, kann die Diffusionskapazität der Lungen mit Prinzipien bestimmt werden, die an und für sich bekannt sind. Durch Studieren der menge eines oder mehrerer löslicher Gase, die im Körper verbleiben ausser der Menge, die sich aus der Verdünnung des alveolären Gases berechnet, kann man den Blutfluss durch die Lungenkapillaren, der in der Regel näherungsweise dem Minutenvolumen des Herzens entspricht, sowie die Flüssigkeitsmenge in den Lungen bestimmen. Die Zufuhr von Testgasimpulsen in unterschiedlichen Abschnitten der Einatmung für verschiedene Atemzüge und das Messen des Verlaufes während nachfolgender Ausatmung ermöglicht ein genaues Studium, wie unterschiedliche Teile des Einatmungsvolumens funktionierende Teile der Lungen erreichen. Aus diesen Daten können Informationen über die Funktion der Luftwege und des Lungenblutkreislaufes gewonnen werden.

Das beschriebene Ausführungsbeispiel des Respirators gemäss der Erfindung kann im Rahmen der folgenden Patentansprüche modifiziert werden, wobei die Vorteile und Möglichkeiten im Hinblick auf die Funktion und die Ausnutzung des Respirators wie oben angegeben beibehalten werden.

**Patentansprüche**

1. Respirator zum Anschließen an die Atemwege von Menschen und Tieren mit einer ersten Anordnung (10) zur Zufuhr und Abgabe von Atemgas an die resp. von den Atemwegen zur Ventilierung der Lungen, mit einer zweiten Anordnung (24) zur ventilgeregelten, separaten, impulsförmigen Zufuhr von Gas zu den Atemwegen unabhängig von dem von der ersten Anordnung verursachten Atemfluß, wobei die zweite Anordnung zum Erzeugen der impulsförmigen Gaszufuhr von Gas zu den Atemwegen unabhängig von dem von der ersten Anordnugn verursachten Atemfluß, wobei die zweite Anordnung zum Erzeugen der impulsförmigen Gaszufuhr mindestens eine Leitung (25) zur Zufuhr von Druckgas und ein daran angeschlossenes Ventil (38a) aufweist, und mit einer Stuereinheit (42) für die zweite Anordnung (24) zum Festlegen mindestens einer charakteristischen Größe der Gasimpulse, dadurch gekennzeichnet, daß die Zufuhr von Atemgas zu den Atemwegen wahlweise von einer der beiden Anordnungen (10, 24) oder durch beide gemeinsam erfolgen kann, wobei für den letzteren Fall die separate impulsförmige Gaszufuhr der zweiten Anordnung (24) während eines wählbaren Zeitabschnittes während des gesamten Atemzyklus erfolgt und daß zusätzlich zu dem vorhandenen Ventil (38a) weitere Ventile (38b-d; 38e-f; 38g-l) an jede Leitung (25, 26, 27) zur Zufuhr von Druckgas parallel angeschlossen sind.

2. Respirator nach Anspruch 1, dadurch gekennzeichnet, dass die Stuereinheit (42) zur Regelung der impulsförmigen Gaszufuhr in Koordination mit der Zufuhr und Abgabe von Atemgas durch die erste Anordnung (10) an diese angeschlossen ist.

3. Respirator nach Anspruch 1 oder 2, dadurch gekennzeichnet, dass jede Leitung (25, 26, 27) in einem Verteiler (32, 33, 34) mit mehreren Anschlüssen mündet, an die eine frei wählbare Anzahl von Ventilen anschliessbar ist.

4. Respirator nach Anspruch 3, dadurch gekennzeichnet, dass alle Anschlüsse jedes Verteilers (32, 33, 34) an ein Ventil angeschlossen sind.

5. Respirator nach einem der Ansprüche 1 bis 4, dadurch gekennzeichnet, dass die Ventile enge Durchlassöffnungen aufweisen.

6. Respirator nach einem de Ansprüche 1 bis 5, dadurch gekennzeichnet, dass ein Druckmessgeber (40) am offenen Ende einer an die erste Anordnung (10) angeschlossenen Trachealkanüle oder in der Nähe davon angeordnet ist und dass dieser Druckmessgeber (40) zum Steuern der ersten (10) und/oder über die Steuereinheit (42) der zweiten (24) Anordnung vorgesehen ist.

7. Respirator nach einem de Ansprüche 1 bis 6, gekennzeichnet durch einen Messaufnehmer (13) zum Erfasen der Gaskonzentrtion im Atemgas zu resp. von den Atemwegen.

8. Respirator nach Anspruch 7, dadurch gekennzeichnet, dass der Messaufnehmer (13) zwischen der ersten Anordnung (10) und der Stelle (21) für die separate ventilgeregelte Gaszufuhr von der zweiten Anordnung (24) in den Atemfluss angeordnet ist.

9. Respirator nach Anspruch 7 oder 8, dadurch gekennzeichnet, dass der Mesaufnehmer (13) zur Steuerung der Mittel (38) für die Erzeugung einer impulsförmigen Gaszufuhr vorgesehen ist.

10. Respirator nach Anspruch 7 oder 8, dadurch gekennzeichnet, dass der Mesaufnehmer (13) zur Steuerung der ersten Anordnung (10) vorgesehen ist.

11. Respirator nach einem der Ansprüche 1 bis 10, dadurch gekennzeichnet, dass die zweite Anordnung (24) einen Befeuchter (22) für die separate ventilgeregelte Gaszufuhr enthält.

12. Respirator nach einem der Ansprüche 1 bis 11, gekennzeichnet durch Mittel zur Zufuhr von pharmakologisch aktiven Substanzen zu den separat zugeführten Gasen.

## Revendications

1. Respirateur destiné à être raccordé aux voies respiratoires d'hommes et d'animaux, comportant un premier dispositif (10) servant à l'envoi du gas respiratoire aux voies respiratoires, et à leur évacuation de ces dernières, pour la ventilation des poumons, un second dispositif (24) servant à réaliser l'envoi séparé, impulsionnel et réglé par une vanne de gaz aux voies respiratoires d'une manière indépendante du flux respiratoire provoqué par le premier dispositif, le second dispositif (24) comportant, pour la production d'un envoi impulsionnel de gaz, au moins une canalisation (25) servant à amener un gaz comprimé et une vanne (38a) raccordée à cette canalisation, et une unité de commande (42) prévue pour le second dispositif (24) afin de fixer au moins une grandeur caractéristique des impulsions de gaz, caractérisé par le fait que l'envoi de gaz respiratoire aux voies respiratoires se fait, au choix, par l'un des deux dispositifs (10, 24) ou par les deux à la fois, étant noté que dans ce dernier cas l'envoi impulsionnel et séparé de gaz par le second dispositif (24) intervient pendant un intervalle de temps à choisir du cycle total de respiration et qu'en plus de la vanne (38a) existante, d'autres vannes (38b-d; 38e-f; 38g-l) sont raccordées en parallèle à chaque canalisation (25, 26, 27) servant à l'envoi du gaz comprimé.

2. Respirateur suivant la revendication 1, caractérisé par le fait que l'unité de commande (42) servant au réglage de l'envoi impulsionnel de gaz d'une manière coordonnée avec l'envoi et l'évacuation du gaz respiratoire au moyen du premier dispositif (10), est raccordée à ce dernier.

3. Respirateur suivant la revendication 1 ou 2, caractérisé par le fait que chaque canalisation (25, 26, 27) débouche dans un répartiteur (32, 33, 34) comportant plusieurs raccords auxquels un nombre pouvant être choisi librement de vanes peuvent être raccordées.

4. Respirateur suivant la revendication 3, caractérisé par le fait que tous les raccords de chaque répartiteur (32, 33, 34) sont raccordés à une vanne.

5. Respirateur suivant l'une des revendications 1 à 4, caractérisé par le fait que les vannes possèdent des ouvertures de passage étroites.

6. Respirateur suivant l'une des revendications 1 à 5, caractérisé par le fait qu'un capteur de mesure de pression (40) est disposé au niveau de l'extrémité ouverte d'une canule trachéale raccordée au premier dispositif (10) ou à proximité de cette canule et que ce capteur de mesure de pression (40) est prévu pour réaliser la commande du premier dispositif (10) et/ou du second dispositif (24), par l'intermédiaire de l'unité de commande (42).

7. Respirateur suivant l'une des revendications 1 à 6, caractérisé par un capteur de mesure (13) servant à déterminer la concentration de gaz dans le gaz respiratoire envoyé aux voies respiratoires ou est évacué de ces dernières.

8. Respirateur suivant la revendication 7, caractérisé par le fait que le capteur de mesure (13) est disposé entre le premier dispositif (10) et l'emplacement (21) prévu pour l'envoi séparé de gaz, réglé par une vanne, à partir du second dispositif (24) dans le flux respiratoire.

9. Respirateur suivant la revendication 7 ou 8, caractérisé par le fait que le capteur de mesure (13) est prévu pour réalise la commande des moyens (38) servant à produire un envoi impulsionnel de gaz.

10. Respirateur suivant la revendication 7 ou 8, caractérisé par le fait que le capteur de mesure (13) est prévu pour réalise la commande du premier dispositif (10).

11. Respirateur suivant l'une des revendications 1 à 10, caractérisé par le fait que le second dispositif (24) contient un humidificateur (22) prévu pour réaliser l'envoi séparé de gaz, réglé par une vanne.

12. Respirateur suivant l'une des revendications 1 à 11, caractérisé par des moyens servant à envoyer des substances actives du point de vue pharmacologique aux gaz envoyés séparément.

**Claims**

1. Respirator for attachment to the airways of humans and animals, having a first arrangement (10) for the supply and emission of respiratory gas to and from the airways for the ventilation of the lungs, having a second arrangement (24) for the valve-controlled, separate, pulsed supply of gas to the airways independently of the respiratory flow produced by the first arrangement, in which respect the second arrangement for generating the pulsed gas supply has at least one line (25), for the supply of pressure gas, and a valve (38a) connected to it, and having a control unit (42) for the second arrangement (24) for fixing at least one characteristic value of the gas pulses, characterized in that the supply of respiratory gas to the airways can be effected alternatively from one of the two arrangements (10, 24) or through both together, in the latter case the separate, pulsed gas supply from the second arrangement (24) taking place during a selectable period of time during the total respiration cycle, and in that, in addition to the existing valve (38a), further valves (38b-d; 38e-f; 38g-l) are connected in parallel to each line (25, 26, 27) for the supply of pressure gas.

2. Respirator according to Claim 1, characterized in that the control unit (42) for regulating the pulsed gas supply in co-ordination with the supply and emission of respiratory gas by the first arrangement (10) is connected to the latter.

3. Respirator according to Claim 1 or 2, characterized in that each line (25, 26, 27) terminates in a distributor (32, 33, 34) having several terminals to which a freely selectable number of valves can be connected.

4. Respirator according to Claim 3, characterized in that all the terminals of each distributor (32, 33, 34) are connected to one valve.

5. Respirator according to one of Claims 1 to 4, characterized in that the valves have narrow through-openings.

6. Respirator according to one of Claims 1 to 5, characterized in that a pressure transducer (40) is arranged at the open end of a tracheal cannula connected to the first arrangement (10), or in the vicinity thereof, and in that this pressure transducer (40) is provided for controlling the first arrangement (10) and/or, via the control unit (42), the second arrangement (24).

7. Respirator according to one of Claims 1 to 6, characterized by a measuring sensor (13) for detecting the gas concentration in the respiratory gas to and from the airways.

8. Respirator according to Claim 7, characterized in that the measuring sensor (13) is arranged between the first arrangement (10) and the position (21) for the separate, valve-controlled gas supply from the second arrangement (24) into the respiratory flow.

9. Respirator according to Claim 7 or 8, characterized in that the measuring sensor (13) is provided for controlling the means (38) for generating a pulsed gas supply.

10. Respirator according to Claim 7 or 8, characterized in that the measuring sensor (13) is provided for controlling the first arrangement (10).

11. Respirator according to one of Claims 1 to 10, characterized in that the second arrangement (24) contains a humidifier (22) for the separate, valve-controlled gas supply.

12. Respirator according to one of Claims 1 to 11, characterized by means for the supply of pharmacologically active substances to the separately supplied gases.

FIG 1

# FIG 2

FIG 3

EP 0 073 219 B2